# EUROPEAN PATENT APPLICATION

(11) **EP 2 388 014 A1**
(43) Date of publication of application: **23.11.2011**
(21) Application number: 10163573.8
(22) Date of filing: 21.05.2010
(51) Int. Cl.: A61K 39/00, C12N 5/0784, A61P 35/02

(54) **Therapeutic application of tumoral immunoglobulin variable region peptides in patients with B-cell malignancies**

(71) Applicant: Dorkoosh, Farid Abedin, 3562 XR Utrecht (NL)
(72) Inventor: Hojjat-Farsangi, Dr Mohammad, Tehran (IR); Rabbani, Dr Hodjattallah, Tehran (IR); Jeddi-Tehrani, Dr Mahmood, Tehran (IR); Shokri, Dr. Fazel, Tehran (IR)
(74) Representative: Johansen, Marianne

(57) **Abstract**

The present invention relates generally to the field of targeted immunotherapy of cancer and, more specifically, to patients with B cell hematologic malignancies such as chronic lymphocytic leukemia (CLL), mantle cell lymphoma, hairy cell leukemia, diffuse large B-cell lymphoma, Burkitt's lymphoma, multiple myeloma and follicular lymphoma. In particular, recombinant or synthetic peptides selected from the variable region of immunoglobulin expressed in leukemic B cells of patients with such malignancies can strongly stimulate tumor-specific T cells (T helper type 1 and T cytotoxic type 1) in these patients. As the nucleotide and amino acid sequences of the variable region of immunoglobulin expressed in leukemic B cells of each patient is specific to that patient, the selected peptides should be designed and constructed specifically for each patient.

## Description

### Field of the invention

The present invention relates generally to the field of targeted immunotherapy of cancer and, more specifically, to patients with B cell hematologic malignancies such as chronic lymphocytic leukemia (CLL), mantle cell lymphoma, hairy cell leukemia, diffuse large B-cell lymphoma, Burkitt's lymphoma, multiple myeloma and follicular lymphoma. In particular, recombinant or synthetic peptides selected from the variable region of immunoglobulin expressed in leukemic B cells of patients with such malignancies can strongly stimulate tumor-specific T cells (T helper type 1 and T cytotoxic type 1) in these patients. As the nucleotide and amino acid sequences of the variable region of immunoglobulin expressed in leukemic B cells of each patient is specific to that patient, the selected peptides should be designed and constructed specifically for each patient.

### Background of the invention

T cells are the most important effector cells in immunosurveillance in normal and also pathological conditions especially against tumor cells (Coulie and van der Bruggen 2003). CD4⁺ T cells are crucial for development of an effective anti-tumor immune response. They provide help to CD8⁺ T cells and natural killer cells by production of IL-2, IFN-γ and TNF-α (Lotze et al. 2000).

T cells recognize peptide antigens that are present on the cell surface in combination with major histocompatibility complex (MHC) antigens (Sredni and Schwartz 1981). Peptides derived from cytoplasmic proteins that are 8 to 11 amino acids in length bind in the groove of class I MHC molecules and are transported via the endoplasmic reticulum to the cell surface. Larger peptides that are derived from the processing of extracellular proteins bind class II MHC molecules and are presented to T cells on the cell surface. Both peptide/MHC-I and peptide/MHC-II complexes are recognized by the T cell receptor (TCR) on CD8 and CD4 T lymphocytes, respectively (Altman et al. 1996).

Tumor-specific T-cell responses can be induced by two different strategies, active or passive immunization. During active immunization (or vaccination), the desired immune response is caused by exposure of the patient's immune system to antigens expressed on tumor cells. The introduction of an immunogenic substance leads to activation and expansion of the endogenous immune repertoire. Passive immunization induces immunity by transfer of preformed tumor-specific T cells (adoptive transfer) (Ivanov et al. 2006). Vaccination is an effective strategy to elicit T-cell responses. Immunotherapeutic strategies are now considered superior in terms of antigen specificity by targeting only tumor cells as opposed to current chemotherapy or radiation therapy that is more general and invasive with many associated side effects (Sundaram et al. 2002). There are several immunotherapeutic strategies that are currently under investigation. Monoclonal antibodies, immune adjuvants, and vaccines against oncogenic viruses are now well-established cancer therapies (Dougan and Dranoff 2009).

Antigens that are expressed at low levels in normal cells and overexpressed in a variety of cancers (tumor associated antigens; TAA) represent an attractive target for immunotherapy. Mostly, these types of proteins are necessary for tumor cells survival, which reduces the probability of immune escape through loss of antigen expression or mutations (Ivanov et al. 2006).

Use of TAA proteins or MHC restricted peptides as vaccines to activate specific T cells is an active area in active immunotherapy of malignancies. For instance, the Wilm's tumor antigen-1 (WT-1) is a zinc finger transcription factor, which is normally expressed in the kidney, testes, ovary, uterus, and lung, and at low levels in normal CD34⁺ hematopoietic cells (Bellantuono et al. 2002; Hirose 1999). WT-1 is aberrantly expressed in both lymphoid and myeloid acute leukemias (Asgarian Omran et al. 2008; Shabani et al. 2008) and contains a number of promising antigenic peptides. WT-1 peptides that bind to HLA-A2 and HLA-A24 have been used to elicit T cells reactive with WT-1 in vitro and in vivo (Bellantuono et al. 2002). WT-1 specific T cells were also able to eliminate leukemic progenitors in immunodeficient mice engrafted with human leukemia, but do not recognize normal CD34⁺ cells (Gao et al. 2000).

Human telomerase reverse transcriptase (hTERT), another example of a TAA, is a component of the ribonucleoprotein complex that is responsible for maintenance of the telomere length and is strongly expressed in most tumors, such as leukemias. Antigen presenting cells (APCs) pulsed with HLA-A2 and HLA-A24 restricted peptides of hTERT have been used to generate T-cell lines and clones that recognize tumor cells expressing high levels of endogenous hTERT (Arai et al. 2001; Vonderheide et al. 1999). Interestingly, these hTERT-specific T cells do not recognize normal hematopoietic cells in vitro (Vonderheide et al. 1999).

Antigens, which are restricted in their pattern of expression to a certain tissue or a differentiation stage, are of special interest for immunotherapy. As immunoglobulins are uniquely expressed on B cells, idiotypic immunoglobulins and CDR3 variable region sequences may be used to elicit immune responses specific to clonal population of leukemic B cells as determined in CLL (Rezvany et al. 2000). However, such individual therapies are not applied routinely in the clinic.

Chronic lymphocytic leukemia (CLL) is the most frequent type of leukemia in the Western world affecting mainly elderly individuals (Dighiero and Hamblin 2008). The malignant B cells in CLL are derived from mature CD5, CD19 and CD23 positive B cells with low expression of surface immunoglobulin (slg). The leukemic B cells of CLL patients mainly accumulate in the bone marrow, blood and lymph nodes (Hallek et al. 2008; Hojjat Farsangi et al. 2008). On the basis of disease progression, CLL has been classified into stable (indolent) and progressive forms.

The expressed immunoglobulin (Ig) in leukemic B cells, like the normal B cells, consists of heavy and light chains. Both the light chains and the heavy chains contain a series of repeating, homologous units, each about 110 amino acid residues in length, that fold independently in a globular motif that is called an Ig domain (Harry W 2008 ). Both heavy chains and light chains consist of amino terminal variable (IGHV and IgVL) regions that participate in antigen recognition and carboxy-terminal constant (CH or CL) regions; the C regions of the heavy chains (CH) mediate effector functions (Harry W 2008).

The variable region of heavy and light chains consists of two different regions; complementarity determining regions or hypervariable regions (CDRs) and framework regions (FRs). Most of the sequence differences among different antibodies are confined to three short sequences in the V region of the heavy chain (heavy chain CDRs: HCDR1, 2, 3) and to three sequences in the V region of the light chain (light chain CDRs: LCDR1, 2, 3). The CDR regions are each about 8-18 amino acid residues long, and they are held in place and separated by more conserved framework sequences (FRs) that make up the Ig domain of the V region. The CDR3 of both IGHV and VL segments are the most variable of the CDRs. Sequence differences among the CDRs of different antibody molecules contribute to distinct interaction surfaces and, therefore, specificities of individual antibodies.

The variable domain of heavy chain is encoded by the rearrangement of three different gene segments referred to as variable (V), diversity (D) and joining (J) gene segments and V and J gene segments for light chain. The V gene solely encodes the CDR1, 2 and FR1, 2, 3 regions of the heavy chain and the CDR3 region is encoded by the V, D and J genes (Harry W 2008).

Some unique epitopes created by the variable region of immunoglobulin expressed on tumor B-cell in B cell malignancies (known as idiotype [Id]) are considered as tumor-specific antigens that can serve as a target for active immunotherapy. Id-vaccines are able to elicit specific immune responses and improve the prognosis of B cell malignancies.

In preclinical studies, vaccination of mice with Id protein coupled to the highly immunogenic carrier protein keyhole limpet hemocyanin (Id-KLH) has been shown to protect animals from subsequent tumor challenge (Campbell et al. 1987; Campbell et al. 1990; George et al. 1988; Kwak et al. 1996; Sugai et al. 1974) and to cure established lymphoma when combined with chemotherapy (Campbell et al. 1988; Kwak et al. 1996).

In humans, different strategies have been employed for Id-vaccination of B cell malignancies. In some types of B cell malignancies this vaccination has no effects, but in others successfully improved treatment of the disease. In some studies, untreated patients with an early stage of multiple myeloma were immunized with autologous alum-precipitated myeloma protein, either with (Osterborg et al. 1998) or without (Bergenbrant et al. 1996) granulocyte-macrophage colony-stimulating factor (GM-CSF). In other studies, Id vaccination was performed with conjugates of Id-keyhole-limpet hemocyanin (Id-KLH) in association with GM-CSF or interleukin-2 (Massaia et al. 1999), or with Id-pulsed dendritic cells (Cull et al. 1999; Lim and Bailey-Wood 1999; Reichardt et al. 1999; Titzer et al. 2000; Wen et al. 1998; Yi et al. 2002). Some of these studies were performed in untreated patients with multiple myeloma, while others were performed after high-dose chemotherapy and stem cell transplantation. Id-specific T-and B-cell responses were detected with variable frequency, but clinical responses were unsatisfactory and not correlated with the induction of tumor-specific immune responses. Thus, it is too early to say whether Id-vaccination and elicitation of Id specific immune responses might improve the prognosis of multiple myeloma patients (Coscia et al. 2004; Rasmussen et al. 2003).

Contrary to multiple myeloma, some vaccine formulations, i.e., Id/KLH conjugates or dendritic cell-based Id vaccines, induced Id-specific immune responses with higher frequency in patients with B-cell lymphoma, with clear evidence of tumor burden reduction and/or improvement of clinical outcome (Bendandi et al. 1999; Timmerman et al. 2002). In two clinical studies, vaccination with ID-KLH plus a chemical adjuvant was found to induce anti-ld immune responses in approximately half the patients with follicular NHL. These immune responses were correlated with improved disease-free survival and overall survival (Hsu et al. 1997; Kwak et al. 1992). The effectiveness of Id-vaccination in B-cell lymphomas is a proof of the validity of Id as a tumor-specific target for activation of immune system.

Apart from the tumor type, which is considered to be important in the success of Id-vaccine immunotherapy, design and formulation of the Id-containing protein or peptide is more relevant. In almost all previous studies, the intact myeloma protein or Fab fragments were used as vaccine for immunization. Basically, the immunizing protein is taken up by APCs and processed to small peptides, which are presented in conjugation with the HLA molecule to autologous T-cells. This process of immunization and antigen presentation has a number of inherent disadvantages, which limits its effectiveness, particularly for CTL activation and Id-targeted cancer immunotherapy. One of the limitations is associated to the outcome of cytoplasmic proteolytic processing of the immunizing protein. Since idiotypic epitopes are restricted to limited sequences of IGHV or IGLV domains, thus enzymatic fragmentation of Ig molecules may fail to produce the target HLA-binding sequence, or produce low levels insufficient for T-cell recognition and activation. The other important issue is preferential presentation of HLA class II-associated peptides due to the extracellular origin of the immunizing myeloma protein. Thus, a strong Id-specific CTL response is usually not expected, though cross presentation could result in CTL activation. Direct loading of APCs by sufficient quantities of pre-selected HLA class I and II-binding peptides together with appropriate adjuvant and a highly immunogenic carrier protein would overcome these limitations leading to induction of a strong Id-specific T helper and CTL responses towards the tumoral cells.

Patients with mutated IGHV and/or IGLV sequences are expected to gain more benefit from this immunotherapeutic invention. Mutated sequences would be recognized as non-self antigens and are regarded as more immunogenic compared to germline sequences.

As the idiotypes expressed on tumoral B cells are weak immunogens, activation of the immune system in patients with B cell malignancies against idiotypes needs adjuvants to be injected in combination with theses idiotypes for vaccination. The importance of Toll-like receptors (TLRs) in stimulating both arms of the immune system, innate and adaptive immunity, is now well known. TLR ligands have thus become interesting targets to use as vaccine adjuvants for cancer treatment. TLR7 and TLR8 were found to be closely related, sharing an intracellular endosomal location, as well as their ligands. Expression of TLR7 and/or TLR8 was found in T cells. In a study by Caron et al. (Caron et al. 2005), T cells were found to be sensitive to resiquimod (R-848) treatment as an agonist of TLR7/8, especially in combination with T-cell receptor (TCR)-dependent and TCR-independent stimuli. This treatment resulted in increased T-cell proliferation and production of gamma interferon and IL-2, with no induction of IL-4 or Th2-attracting chemokines (Caron et al. 2005). Moreover, it was found that memory T cells were more sensitive to this TLR-mediated activation than naïve T cells. In general, administration of TLR7/8 agonists results in activation of CD4+ Th1 cells and enhanced CD8+ T-cell responses (Ramakrishna et al. 2007; Smorlesi et al. 2005; Thomsen et al. 2004), at least partially mediated by the induced Th1-polarizing cytokine pattern (Smith et al. 2004; Wagner et al. 1999). TLR7/8 agonists lead to better priming of CTLs by efficient maturation and activation of APCs (Ramakrishna et al. 2007; Rechtsteiner et al. 2005), and CD8+ T cells showed a higher expression level of perforin and cytotoxic granule markers (Ambach et al. 2004; Smith et al. 2004).

Beside TLR7/8 agonists, TLR9 agonists, referred to as CpG oligodeoxynucleotides (CpG ODN) have demonstrated substantial potential as vaccine adjuvants, and as mono- or combination therapies for the treatment of cancer and infectious and allergic diseases. CpG ODN, as the agonists of TLR9, enhance the development of antigen-specific CD4 and CD8 T cell responses as an indirect consequence presumably by the induction of Th1-like cytokine and chemokine milieu and DC maturation and differentiation, which may explain the resulting strong CTL generation, even in the absence of CD4 T cell help (Lipford et al. 2000; Sparwasser et al. 2000).

In patients with CLL, based on nucleotide sequence homology of the IGHV gene expressed in the leukemic B cells, to the closest IGHV germline gene, patients are classified into mutated and unmutated groups (Fais et al. 1998). This classification was first proposed by Fais et al. in 1998 (Fais et al. 1998) and was confirmed by other groups (Damle et al. 1999; Hamblin et al. 1999; Hamblin et al. 2000). In the mutated group of patients, the IGHV gene expressed on the leukemic B cells has more than 2% mutation in its sequence (less than 98% homology with the germline IGHV gene), while in the unmutated group the frequency of mutations in IGHV gene is less than 2% (more than 98% homology with germline IGHV gene) (Fais et al. 1998).

Molecular or immunological tools for determining the prognosis of CLL patients have not been developed.

### Summary of the invention

In a first embodiment the invention provides a recombinant or synthetic 9-20 amino acid length peptide having amino acid sequence identity with an amino acid sequence selected from an expressed immunoglobulin variable region of leukemic B cells, wherein said B cells are derived from a patient with a B cell malignancy, wherein said variable region is either: the variable region of a IGHV consisting of an amino acid sequence starting at the first amino acid of framework 1 and ending at the last amino acid of HCDR3; or the variable region of a IGLV consisting of an amino acid sequence starting at the first amino acid of framework 1 and ending at the last amino acid of LCDR3.

In a further embodiment the invention provides autologous dendritic cells pulsed with the recombinant or synthetic 9-20 amino acid length peptide of the invention.

In a further embodiment the invention provides a pharmaceutical composition comprising the recombinant or synthetic 9-20 amino acid length peptide, or the autologous dendritic cells of the invention.

In a further embodiment the invention provides a vaccine comprising the recombinant or synthetic 9-20 amino acid length of the invention for treatment of patients diagnosed with a B-cell malignancy.

In a further embodiment the invention provides a method of immunization of patients with B-cells malignancies (e.g. CLL) with the recombinant or synthetic peptide, or the pharmaceutical composition of the invention for treatment of patients diagnosed with a B-cell malignancy.

### Detailed description of the invention

### Brief description of the drawings

Figure 1 is a graph illustrating that the frequency of autologous T cells secreting IFN-γ against IGHV and CDR3 peptides are significantly higher in indolent CLL patients compared to progressive CLL patients.
Figure 2 is a graph illustrating that the frequency of autologous T cells secreting IFN-γ against IGHV peptides are significantly higher in IGHV mutated CLL patients compared to unmutated CLL patients.
Figure 3 is a graph illustrating that the frequency of autologous T cells secreting IFN-γ against IGHV peptides are significantly higher in IGHV mutated/indolent CLL patients compared to unmutated/progressive CLL patients.
Figure 4 is a graph illustrating that the amount of IFN-γ secreted by autologous T cells against IGHV peptides are significantly higher in IGHV mutated CLL patients compared to unmutated CLL patients.
Figure 5 is a graph illustrating that the proliferation of autologous T cells induced by IGHV peptides is higher in IGHV mutated CLL patients compared to unmutated CLL patients.

### I. Immunotherapeutic peptides for use in the treatment of cancer

Experimental works during the last decades established that T cell immunity can limit tumor progression and also mediates tumor rejection. In this regard, activation of tumor specific cytotoxic T cells is the goal of most immunotherapeutic strategies against tumor cells. In a few cancers, tumor cells express tumor specific antigens (TSA) that can specifically activate T cells. Tumor cells usually express tumor associated antigens (TAA) to which, with a few exceptions, vigorous antigen-specific T cell response is not normally induced. In B cell malignancies, immunoglobulin expressed on tumoral B cells can activate specific T cells. In the present invention we show that recombinant or synthetic peptides based on the variable region of the heavy (IGHV) and light chain (IGLV) of the immunoglobulin expressed in patient's leukemic B cells can strongly activate autologous T cells against tumoral B cells in patients with B cell malignancies. As the amino acid sequence of immunoglobulin variable region expressed in leukemic B cells in patients with B cell malignancies are different from each other, these peptides should be designed and constructed individually for each patient. Self HLA-binding capability of selected peptides is essential for their immunogenicity and therapeutic effectiveness. This strategy is more useful for patients with mutations in the sequence of immunoglobulin variable region expressed on their leukemic B cells, since introduction of mutations in the germline sequence, results in formation of new or more immunogenic epitopes for activation of autologous T cells. Use of such epitopes constructed as recombinant or synthetic peptides strongly activates cytotoxic T cells against tumoral B cells in these patients.

More specifically the present invention is based on the recognition that mutations play a key role in the prognosis of CLL patients.

In one embodiment the invention provides a method for the selection of the mutated 9-20 amino acid peptides from the immunoglobulin heavy and light chain variable region sequence expressed on the leukemic B cells of patients with B cell malignancies for effective activation of autologous T cells.

In a further embodiment the present invention provides a method to design the CDR3 sequence of CLL patients that can strongly activate specific helper and cytotoxic T cells, leading to potentiation of the tumor specific immune response and improvement of disease progression in treated patients.

### Example 1: Samples and subjects

The methods described herein can be applied to the peripheral blood mononuclear cells (PBMC), preferably purified B cells from patients with B cell malignancies such as chronic lymphocytic leukemia, mantle cell lymphoma, hairy cell leukemia, diffuse large B-cell lymphoma, Burkett's lymphoma and follicular lymphoma.

Typically the methods used herein are used to determine information regarding a subject, or to determine a relationship between nucleic acid sequence mutation and disease outcome. The samples used in the methods described herein are selected according to the purpose of the method to be applied. For example, samples can contain nucleic acid molecules (e.g. complimentary DNA-cDNA) encoding immunoglobulin heavy chain variable region expressed by malignant B cells, whose nucleic acid sequence can be used to correlate mutations with the presence or absence of disease progression. In another example, samples can contain nucleic acid molecules (e.g. complimentary DNA-cDNA) encoding heavy chain CDR3 of leukemic B cells from one individual, where the sample is examined to determine the disease state or tendency towards disease of the individual. One skilled in the art can use the methods described herein to determine the desired sample to be examined.

In one embodiment, samples can contain nucleic acid molecules (e.g. complimentary DNA-cDNA) encoding the immunoglobulin light chain variable region expressed in malignant B cells, whose nucleic acid sequence can be used to correlate mutations with the presence of disease progression. In one embodiment, samples can contain nucleic acid molecules (e.g. complimentary DNA-cDNA) encoding light chain CDR3 expressed by leukemic B cells from one individual, where the sample is examined to determine the disease state or tendency towards disease of the individual.

A sample may be from any patients with mutated or unmutated sequence in the expressed immunoglobulin heavy or light chain variable region on leukemic B cells; for example patients with B cell malignancies, including for example, chronic lymphocytic leukemia, mantle cell lymphoma, hairy cell leukemia, diffuse large B-cell lymphoma, Burkitt's lymphoma and follicular lymphoma.

### Example 2: RNA and DNA isolation

Total cellular RNA was isolated from 3 to 5 million PBMCs of patients using RNA-Bee (TEL- TEST Inc, USA) based on the guanidine thiocyanate- phenol-chloroform extraction method (Chomczynski and Sacchi 1987). High molecular weight DNA was extracted from 10 million PBMCs of patients by the phenol-chloroform extraction method (Olerup and Zetterquist 1992). The concentration of the corresponding RNA or DNA was measured by spectrophotometry.

### Example 3: Complementary DNA (cDNA) synthesis and PCR amplification of IGHV or IGLV (Vk and Vλ) region

First strand cDNA was synthesized from 3 microgram of total RNA (unfolded at 90°C for 5 min) using M-MuLV reverse transcriptase (RT) enzyme, random hexamer primers (20 pmol) and dNTPs (20 mM). The mixture was incubated at 42 °C for 45 min followed by one minute at 90 °C for inactivation of RT enzyme.

Serial dilutions of DNA and cDNA (1:10 to 1:5000) were prepared and PCR amplification was performed using IGHV or IGLV (Vk and Vλ) family specific degenerated primers (see Tables 1-3 where S designates a "forward sense primer" and AS designates a "reverse antisense primer").

Table 1 represents the sequence of human IGHV family specific primers used for amplification of the expressed immunoglobulin heavy chain variable region in leukemic B cells of patients with B cell malignancies.

**Table 1. Sequence of human IGHV family specific primers**

| **Primer** | **sequence** | **Amplicon size (bp)** |
|---|---|---|
| **IGHV1 family (LS)(S)** | 5'- ATGGACTGSACCTGGAGVRTCYTC -3' | |
| **IGHV2 family (LS) (S)** | 5'- ATGGACACACTTTGCTMCACRCTC -3' | |
| **IGHV3a family (LS) (S)** | 5'- ATGGARYTKKGRCTSMGCTGGVTTT -3' | |
| **IGHV3b family (LS) (S)** | 5'-ATGGARTTKKGRCTKWGCTGGGTTT-3' | All ~ 420 |
| **IGHV4 family (LS) (S)** | 5'- ATGAARCAYCTGTGGTTYTTCCTYC -3' | |
| **IGHV5 family (LS) (S)** | 5'- ATGGGGTCAACCGCCATCCTYGSC -3' | |
| **IGHV6 family (LS) (S)** | 5'- ATGTCTGTCTCCTTCCTCATCTTCC -3' | |
| **IGHV7 family (LS) (S)** | 5'- ATGGACTGGACCTGGAGGATCCTC -3' | |
| **IGHJ1-3 (AS)** | 5'-TTGAGGAGACRGTGACCAGGGTKCCMYGGCC-3' | |
| **IGHJ4-5 (AS)** | 5'- TTGARGAGACGGTGACCGTKGTCCCTTGGCC-3' | |
| **IGHJ (AS)** | 5'- TTGARGAGACRGTGACCRKKGT -3' | |

Table 2 represents the sequence of human kappa light chain family specific primers used for amplification of the expressed immunoglobulin light chain in leukemic B cells of patients with B cell malignancies.

**Table 2. Sequence of human Ig Vk family specific primers**

| **Primer** | **sequence** | **Amplicon size (bp)** |
|---|---|---|
| **IGVK I-LS (S)** | 5'- ATRGACATGAGRGTSCYYGCTCAG -3' | |
| **IGVK II-LS (S)** | 5'- ATGAGGCTCCYTGCTCAGCTYCTG -3' | |
| **IGVK III-LS (S)** | 5'- ATGGAARCCCCAGCKCAGCTTCTC -3' | |
| **IGVK IV-LS (S)** | 5'- ATGGTGTTGCAGACCCAGGTCTTCA -3' | All -600 |
| **IGVK V-LS (S)** | 5'- ATGGGGTCCCAGGTTCACCTCCTC -3' | |
| **IGVK VI-LS (S)** | 5'- ATGTTGCCATCACAACTCATTGGG -3' | |
| **HIGKC (AS)** | 5'- CTAGCACACACCCCTGTTGAAGCTGTT -3' | |

Table 3 represents the sequence of human lambda light chain family specific primers used for amplification of the expressed immunoglobulin light chain in leukemic B cells of patients with B cell malignancies.

**Table 3. Sequence of human Ig Vλ family specific primers**

| **Primer** | **sequence** | **Amplicon size (bp)** |
|---|---|---|
| **VL1-S** | 5'-GTGYTGACKCAGCCRCCCTCRGYG -3' | All -300 |
| **VL2-S** | 5'- GCCCTGACTCAGCCTSSCTCMGYG -3' | |
| **VL3-S** | 5'- GWGCTGAYDCAGSHMYYYKCDGTG -3' | |
| **VL4-S** | 5'- GTGCTGACTCARYCVYCSTCTGCM -3' | |
| **VL5-S** | 5'- GTGCTGACTCAGCCRBCTTCCYHY -3' | |
| **VL6-S** | 5'- ATGCTGACTCAGCCCCACTCTGTG -3' | |
| **VL7-S** | 5'-GTGGTGACTCAGGAGCCCTCACTG -3' | |
| **VL8-S** | 5'- GTGGTGACCCAGGAGCCATCGTTC -3' | |
| **VL9-S** | 5'- GTGCTGACTCAGCCACCTTCTGCA -3' | |
| **VL10-S** | 5'- GGGCTGACTCAGCCACCCTCGGTG -3' | |
| **HIGLC-AS** | 5'- CTATGAACATTCYGYAGGGGCCACTGT -3' | |

Polymerase chain reaction (PCR) was performed in 35 cycles, initiated by 5 min at 94 °C followed by 94 °C for 45 second and 72 °C for 45 second and 10 min at 72 °C for the final extension. PCR products were finally visualized by electrophoresis on 1.5% agarose gel containing ethidium bromide. The length of the nucleic acid target gene molecule that may be used in the current methods may vary according to the sequence of the nucleic acid target gene molecule.

### Example 4: HLA typing

DNA was used for HLA typing of patients. DNA typing for HLA-A, B, DRB and DQB alleles was performed in patients using a sequence-specific primer polymerase chain reaction (SSP-PCR) technique following the method of Olerup and Zetterquist (Olerup and Zetterquist 1992). HLA-A, B, DRB and DQB low-resolution kits were supplied by Qiagen Vertriebs GmbH (Vienna, Austria). Genomic DNA was amplified by 23 PCR reactions for HLA-A, 47 reactions for HLA-B and 31 reactions for HLA-DRB-DQB1 specificities. Each PCR reaction was performed on 30 ng of extracted DNA using 0.5 U of Taq DNA polymerase (Fermentas Life Science, Ontario, Canada), 3µl of master mix containing oligonucleotides (200 µmol/L), PCR buffer (50 mmol/L KCI, 1.5 mmol/L MgCl2, 10 mmol/L Tris-HCl, pH 8.3, 0.001% w/v gelatin), 5% glycerol, and 100 µg/ml Cresol red. Each well contained group-specific primer pairs with a different product size and internal positive control primer pairs. An initial denaturation step at 94°C for 2 minutes was followed by 10 two-temperature cycles (94°C for10 seconds and 65°C for 60 seconds) and 20 three-temperature cycles (94°C for 10 seconds, 61°C for 50 seconds, and 72°C for 30 seconds). The detection of amplified alleles was carried out by electrophoresis of PCR products on a 2% (w/v) agarose gel in 0.5X TBE buffer containing 5 µl (10 mg/ml) ethidium bromide solution per 100 ml of gel.

### Example 5: Sequencing of PCR products and analysis of Ig gene sequences for mutations

Clonal PCR products (e.g. PCR products of example 3) were purified by gel extraction kit. Gel purified PCR products were cloned into pGEM-T easy vector (Promega, UK). Briefly, after transformation of chemically prepared JM109 competent cells, at least five colonies were selected randomly for sequencing. Sequencing was performed from both directions using the BigDye Terminator Cycle Sequencing Reaction Kit (Applied Biosystems, USA), T7 and SP6 primers. For each sample IGHV, IgDH, and IgJH genes and presence of mutations (encoding amino acid mutations) were identified by matching to the closest known human germline gene using the ImMuno-Gene Tics (IMGT) Database (http://imgt.cines.fr) and the IgBLast search.

### Example 6: Peptide design

Based on the deduced amino acid sequence of the immunoglobulin heavy or light chain variable regions expressed in the leukemic B cells of patients, 9 to 20 mer HLA-A and HLA-DR matched peptides were synthesized. Peptides were synthesized based on the original amino acid sequence of the expressed immunoglobulin in the leukemic B cells of patients with B cell malignancies. In one embodiment, the peptides are selected from the mutated part of the expressed immunoglobulin heavy chain variable region (framework region 1 to framework region 3 (FWR1-FWR3) in which the mutations take place in the middle of the peptides. In one embodiment, the peptides are selected from the sequence of the expressed immunoglobulin heavy chain variable region (FWR1-FWR3) with no mutation and high-moderate affinity for HLA-A and HLA-DR of patients with B cell malignancies. In one embodiment, the peptides are selected from the mutated sequence of the expressed immunoglobulin light chain variable region (FWR1-FWR3) in which the mutations take place in the middle of the peptides. In one embodiment, the peptides are selected from the sequence of the expressed immunoglobulin light chain variable region (FWR1-FWR3) with no mutation and with the high-moderate affinity for HLA-A and HLA-DR of patients with B cell malignancies. In one embodiment, the peptides are selected from the CDR3 region of the immunoglobulin heavy chain variable region expressed in the leukemic B cells of patients with B cell malignancies. In one embodiment, the peptides are selected from the CDR3 region of the immunoglobulin light chain variable region expressed in the leukemic B cells of patients with B cell malignancies.

The affinity of designed peptides for binding to HLA-A and HLA-DR of patients was examined by SYFPEITHI database (http://www.syfpeithi.de/Scripts/MH CServer.dII /EpitopePrediction.htm) and ProPred-I (http://www.imtech.res.in/raghava/propred1/) and other reliable online databases.

### Example 7: Production of recombinant IGHV or IGLV peptides in prokaryotic and eukaryotic cells

Selected 9-20 amino acid (27-75 nucleotides) encoding nucleic acid molecules are amplified using specific flanking primers designed based on the complete nucleotide sequence of the target IGHV or IGLV gene of the patient's leukemic B-cells. Sequences of restriction enzymes (e.g. EcoR1 and Not 1) are added to the designed primers for cloning the amplified PCR products. Amplified PCR products encoding an IGHV or IGLV sequence derived from leukemic B cells of each patient are cloned in pGEM-T easy vector, and excised by EcoRI and Not I restriction enzymes and then cloned into pGAPz alpha-A and pcDNA3.1 expression vectors. After insertion of the selected sequences into the vectors, the constructed plasmids are transformed into Origami (bacteria), Pichia pastoris (yeast) or HEK293 human cell line. Stable clones secreting the recombinant peptide, preferably as fusion proteins, e.g. His-tag fusion protein, are selected and cultured in the desired culture media. Secreted recombinant IGHV or IGLV peptides are then purified from the supernatant of cell culture through their affinity tag, e.g. His tag using Ni columns.

### Example 8: Monocyte and T cell enrichment from peripheral blood mononuclear cells

For each patient, monocytes were purified from peripheral blood mononuclear cells (PBMC) by magnetic bead cell separation using Miltenyi biotec positive selection kits (Miltenyi Biotec, USA). Briefly, PBMCs were isolated and mixed with anti CD14 antibody coated MACS microbeads. CD14 positive cells were enriched by positive selection method using midiMACS columns. The purity of cells was >95% as determined by flowcytometry.

T cells were purified from each patient by magnetic bead cell separation using Miltenyi biotec negative selection kits. Briefly, purified PBMC were mixed with a cocktail of antibodies containing antibodies to CD14, CD16, CD19, CD36, CD56, CD136 and glycophorine A. After incubation and washing, cells were mixed and incubated with anti-biotin antibodies coated MACS microbeads. Finally, T cells were enriched by negative selection method using midiMACS columns. The purity of cells was >98% as determined by flow cytometry.

### Example 9: Dendritic cell (DC) generation from peripheral blood monocytes

Dendritic cells were generated from purified monocytes (Romani et al. 1996). IL-4 (20 ng/ml) (Peprotech England) and GM-CSF (50 ng/ml) were added to cultured monocytes for 5 days to produce immature DCs. On the fifth day peptides (10 µg/ml) were added to immature DCs. Next day, TNF-α (20 ng/ml) and poly-IC (50 µg/ml) (Invivogen, USA) were added to immature DCs. Mature DCs were harvested on the 7^{th} day to be used in T cell response assays. Peptide-loaded mature DCs were also employed for vaccination of the same patients. In one embodiment, DC vaccination is accompanied by administration of a cocktail of selected peptides with or without adjuvant, as described later.

### Example 10: T cell response assays

Autologous T cells and mature dendritic cells loaded with peptides were mixed and the T cell response against loaded peptides on dendritic cells was measured after 48 hours, by three methods including ELISPOT, ELISA and LTT.

ELISPOT assay was used to **check** the frequency of T cells secreting IFN-γ (Mabtech, Sweden) against peptides from IGHV and CDR3 peptides. Briefly ELISPOT plates (Millipore) were coated with monoclonal antibody against IFN-y (mAb-D1K) (1 µg/ml in PBS), incubated overnight at 4°C, washed with PBS to remove unbound antibody, and blocked with RPMI 1640 containing 10% human AB serum for 2 hour at 37°C. Purified T cells (>98% pure) were plated with autologous dendritic cells loaded with peptides (E: APC ratio, 10:1) and incubated for 48 hours at 37°C. Negative control wells contained APCs and T cells without peptides or with irrelevant peptides (HIV RT enzyme).

Phytohemagglutinin (PHA, Sigma) at a concentration of 5 µg/ml, Protein purified derivative (PPD) and a sequence of Influenza matrix protein (10 µg/ml) were used as positive controls.

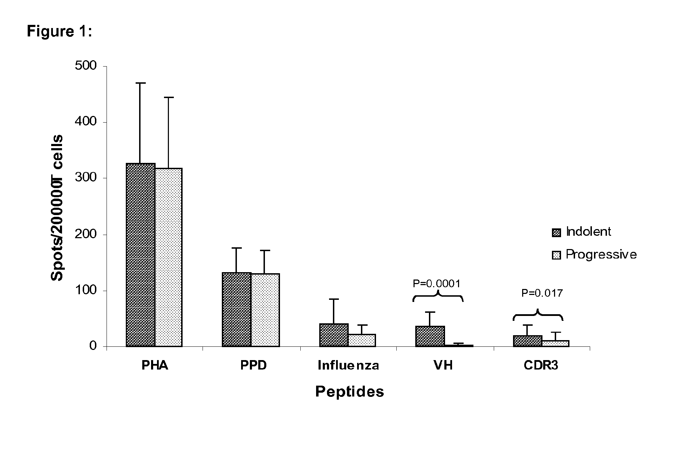

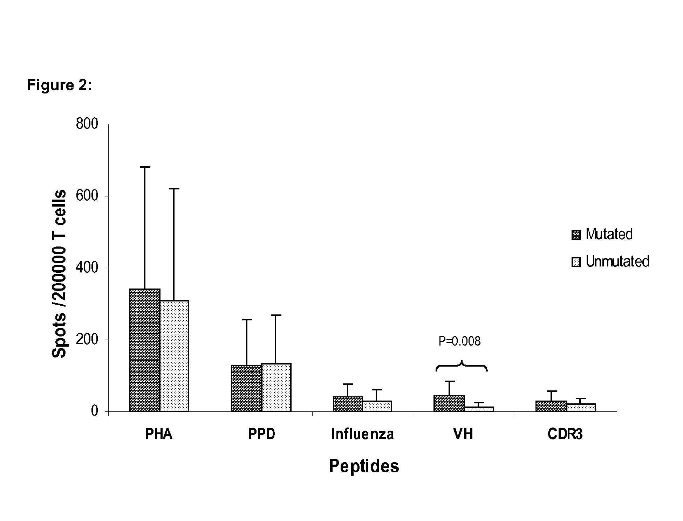

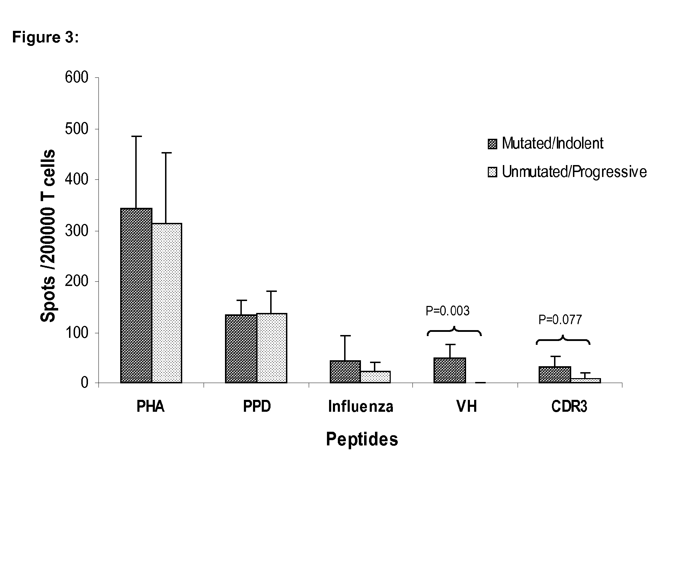

After 48 hours incubation, T cell supernatants were removed for ELISA assay, and after washing, biotinylated detection antibody against human IFN-γ (1 µg/mL; Mab7-B6-1; Mabtech) was added. Plates were incubated for an additional 2 h at 37°C. After incubation and washing, Streptavidin-ALP (1/1000) was added and incubated for 1 hour at room temperature (RT) and finally spot development was done after addition of substrate and spots were counted manually. Representative results are presented in figures 1-3. The frequency of autologous T cells secreting IFN-y against IGHV and CDR3 peptides were significantly higher in indolent CLL patients compared to progressive CLL patients (figure 1). The frequency of autologous T cells secreting IFN-y against IGHV peptides are significantly higher in IGHV mutated CLL patients compared to unmutated CLL patients (figure 2). The frequency of autologous T cells secreting IFN-y against IGHV peptides are significantly higher in IGHV mutated/indolent CLL patients compared to unmutated/progressive CLL patients (figure 3).

No statistically significant differences were observed for positive controls (PHA, PPD and Influenza peptides) between indolent vs progressive, IGHV mutated vs unmutated and IGHV mutated/indolent CLL patients compared to unmutated/progressive CLL patients.

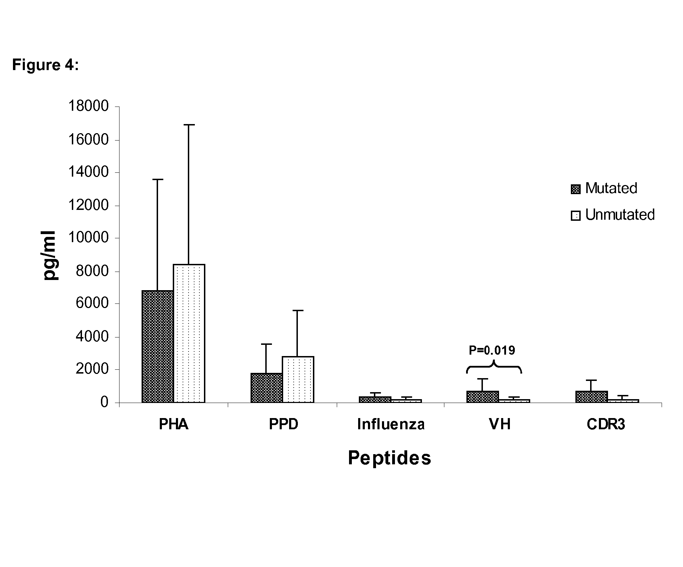

Soluble IFN-y was detected by ELISA assay (Peprotech, England) in the supernatant of T cells culture in ELISPOT assay. Briefly, ELISA plates (Nunc) were coated with monoclonal antibody against IFN-y (2µg/ml in PBS; Peprotech, England), incubated overnight at RT, washed with PBS to remove unbound antibody, and blocked with PBS containing 1 % BSA for 1 hour at RT. Then plates were washed and T cell supernatants were added to the ELISA wells, incubated for 2 hours at RT. After washing, biotinylated detection antibody against human IFN-y (0.5 µg/mL; Peprotech, England) was added and incubated for 2 hours at RT. Finally, the optical density (OD) of ELISA wells was measured after addition of enzyme conjugate and substrate (Peprotech, England). An example of the results is shown in figure 4. The amount of IFN-y secreted by autologous T cells against IGHV peptides was significantly higher in IGHV mutated CLL patients compared to unmutated CLL patients (Figure 4).

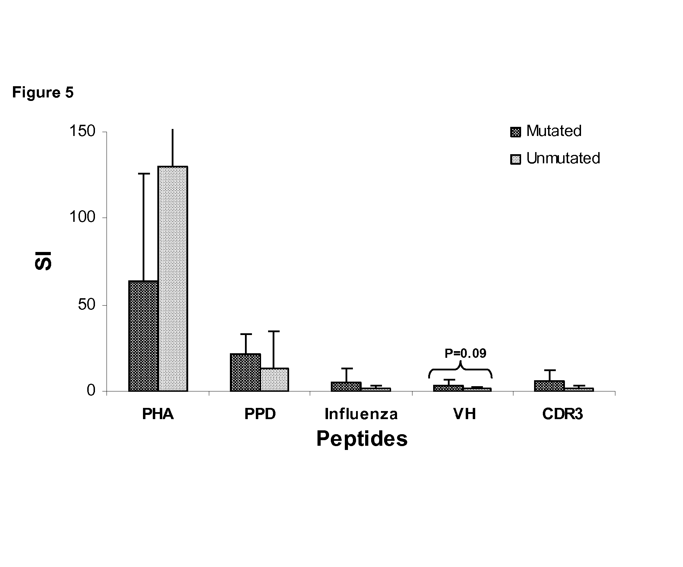

Proliferation of T cells against IGHV and CDR3 peptides was measured by conventional lymphocyte transformation test (LTT) assay. This method is known to one skilled in the art. In this assay negative control wells contained APCs and T cells without peptides or with irrelevant peptides (HIV RT enzyme) (10 µg/ml). Phytohemagglutinin (PHA, Sigma) at a concentration of 5 µg/ml, Protein purified derivative (PPD) and a sequence in Influenza matrix protein (10 µg/ml) were used as positive controls. Incorporated radioactive thymidine was measured by a Beta counter and presented as stimulation index (SI). An example of the results is shown in figure 5. The proliferation of autologous T cells induced by IGHV peptides is higher in IGHV mutated CLL patients compared to unmutated CLL patients (Figure 5).

### Example 11: Composition of the peptide vaccine for vaccination of patients

A combination of 2-4 synthetic peptides (described in example 6) (patients HLA matched) with 9-20 amino acids length (e.g. one 9 mer from IGHV or IGLV sequence, one 9 mer from CDR3 sequence, one 15 mer from IGHV and/or IGLV sequence and one 9-20 mer from CDR3 sequence) with GMP grade (>98% purity and endotoxin free) are dissolved in 20 mM of phosphate-buffered saline (pH 7.5) and emulsified with an adjuvant (e.g. GM-CSF, CpG, R848 or MPL); the final ratio of phosphate-buffered saline to adjuvant by volume is 50:50. One dose of each vaccine may contain 0.2-0.6 mg of each peptide in a total volume of 1.5 ml. Each patient is vaccinated intramuscularly (im) with these self peptides four times at 2-3 weeks time intervals. The peptides may be used with or without conjugation to a potent T-cell stimulator such as tetanus toxoid.

In another aspect the combination of peptides are loaded on patients dendritic cells (as described in example 9). All eligible patients are scheduled for at least 4 biweekly vaccinations with 10⁷-10⁹ autologous mature peptide pulsed dendritic cells together with appropriate adjuvant. From the 2^{nd} vaccine dose, concomitantly IFN-γ or IL-2 could be administered to boost the CTL immune response.

### Example 12: Composition of the recombinant vaccine for vaccination of patients

A combination of 2-4 IGHV and/or IGLV recombinant peptides (described in example 7) are dissolved in 20 mM of phosphate-buffered saline (pH 7.5) and emulsified with an appropriate adjuvant (e.g. GM-CSF, CpG, R848 or MPL); the final ratio of phosphate-buffered saline to adjuvant by volume is 50:50. One dose of each vaccine contains 0.2-0.6 mg of each peptide in a total volume of 1.5 ml. Each patient is vaccinated im with these self recombinant peptides four times at 2-3 weeks time intervals.

In another aspect the combination of recombinant peptides are loaded on patients dendritic cells (as described in example 9). All eligible patients are scheduled for at least 4 biweekly vaccinations with 10⁷-10⁹ autologous mature recombinant peptide pulsed dendritic cells.

### References:

Altman JD, Moss PA, Goulder PJ, Barouch DH, McHeyzer-Williams MG, Bell JI, McMichael AJ and Davis MM (1996) Phenotypic analysis of antigen-specific T lymphocytes. Science 274: 94-6.
Ambach A, Bonnekoh B, Nguyen M, Schon MP and Gollnick H (2004) Imiquimod, a Toll-like receptor-7 agonist, induces perforin in cytotoxic T lymphocytes in vitro. Mol Immunol 40: 1307-14.
Arai J, Yasukawa M, Ohminami H, Kakimoto M, Hasegawa A and Fujita S (2001) Identification of human telomerase reverse transcriptase-derived peptides that induce HLA-A24-restricted antileukemia cytotoxic T lymphocytes. Blood 97: 2903-7.
Asgarian Omran H, Shabani M, Vossough P, Sharifian R, Tabrizi M, Khoshnoodi J, Jeddi-Tehrani M, Rabbani H and Shokri F (2008) Cross-sectional monitoring of Wilms' tumor gene 1 (WT1) expression in Iranian patients with acute lymphoblastic leukemia at diagnosis, relapse and remission. Leuk Lymphoma 49: 281-90.
Bellantuono I, Gao L, Parry S, Marley S, Dazzi F, Apperley J, Goldman JM and Stauss HJ (2002) Two distinct HLA-A0201-presented epitopes of the Wilms tumor antigen 1 can function as targets for leukemia-reactive CTL. Blood 100: 3835-7.
Bendandi M, Gocke CD, Kobrin CB, Benko FA, Sternas LA, Pennington R, Watson TM, Reynolds CW, Gause BL, Duffey PL, Jaffe ES, Creekmore SP, Longo DL and Kwak LW (1999) Complete molecular remissions induced by patient-specific vaccination plus granulocyte-monocyte colony-stimulating factor against lymphoma. Nat Med 5: 1171-7.
Bergenbrant S, Yi Q, Osterborg A, Bjorkholm M, Osby E, Mellstedt H, Lefvert AK and Holm G (1996) Modulation of anti-idiotypic immune response by immunization with the autologous M-component protein in multiple myeloma patients. Br J Haematol 92: 840-6.
Campbell MJ, Carroll W, Kon S, Thielemans K, Rothbard JB, Levy S and Levy R (1987) Idiotype vaccination against murine B cell lymphoma. Humoral and cellular responses elicited by tumor-derived immunoglobulin M and its molecular subunits. J Immunol 139: 2825-33.
Campbell MJ, Esserman L, Byars NE, Allison AC and Levy R (1990) Idiotype vaccination against murine B cell lymphoma. Humoral and cellular requirements for the full expression of antitumor immunity. J Immunol 145: 1029-36.
Campbell MJ, Esserman L and Levy R (1988) Immunotherapy of established murine B cell lymphoma. Combination of idiotype immunization and cyclophosphamide. J Immunol 141: 3227-33.
Caron G, Duluc D, Fremaux I, Jeannin P, David C, Gascan H and Delneste Y (2005) Direct stimulation of human T cells via TLR5 and TLR7/8: flagellin and R-848 up-regulate proliferation and IFN-gamma production by memory CD4+ T cells. J Immunol 175: 1551-7.
Chomczynski P and Sacchi N (1987) Single-step method of RNA isolation by acid guanidinium thiocyanate-phenol-chloroform extraction. Anal Biochem 162: 156-9.
Coscia M, Mariani S, Battaglio S, Di Bello C, Fiore F, Foglietta M, Pileri A, Boccadoro M and Massaia M (2004) Long-term follow-up of idiotype vaccination in human myeloma as a maintenance therapy after high-dose chemotherapy. Leukemia 18: 139-45.
Coulie PG and van der Bruggen P (2003) T-cell responses of vaccinated cancer patients. Curr Opin Immunol 15: 131-7.
Cull G, Durrant L, Stainer C, Haynes A and Russell N (1999) Generation of anti-idiotype immune responses following vaccination with idiotype-protein pulsed dendritic cells in myeloma. Br J Haematol 107: 648-55.
Dighiero G and Hamblin TJ (2008) Chronic lymphocytic leukaemia. Lancet 371: 1017-29.
Dougan M and Dranoff G (2009) Immune therapy for cancer. Annu Rev Immunol 27: 83-117.
Fais F, Ghiotto F, Hashimoto S, Sellars B, Valetto A, Allen SL, Schulman P, Vinciguerra VP, Rai K, Rassenti LZ, Kipps TJ, Dighiero G, Schroeder HW, Jr., Ferrarini M and Chiorazzi N (1998) Chronic lymphocytic leukemia B cells express restricted sets of mutated and unmutated antigen receptors. J Clin Invest 102: 1515-25.
Gao L, Bellantuono I, Elsasser A, Marley SB, Gordon MY, Goldman JM and Stauss HJ (2000) Selective elimination of leukemic CD34(+) progenitor cells by cytotoxic T lymphocytes specific for WT1. Blood 95: 2198-203.
George AJ, Folkard SG, Hamblin TJ and Stevenson FK (1988) Idiotypic vaccination as a treatment for a B cell lymphoma. J Immunol 141: 2168-74.
Hallek M, Cheson BD, Catovsky D, Caligaris-Cappio F, Dighiero G, Dohner H, Hillmen P, Keating MJ, Montserrat E, Rai KR and Kipps TJ (2008) Guidelines for the diagnosis and treatment of chronic lymphocytic leukemia: a report from the International Workshop on Chronic Lymphocytic Leukemia updating the National Cancer Institute-Working Group 1996 guidelines. Blood 111: 5446-56.
Hamblin TJ, Orchard JA, Gardiner A, Oscier DG, Davis Z and Stevenson FK (2000) Immunoglobulin V genes and CD38 expression in CLL. Blood 95: 2455-7.
Harry W SJ (2008 ) Ilmmunoglobulins: Structure and Function. In: Paul W (ed) Fundamental Immunology, pp 127-151, Lippincott Williams & Wilkins Hirose M (1999) The role of Wilms' tumor genes. J Med Invest 46: 130-40.
Hojjat Farsangi M, Jeddi-Tehrani M, Razavi SM, Sharifian RA, Shamsian Khoramabadi A, Rabbani H and Shokri F (2008) Immunophenotypic characterization of the leukemic B-cells from Iranian patients with chronic lymphocytic leukemia: association between CD38 expression and disease progression. Iran J Immunol 5: 25-35.
Hsu FJ, Caspar CB, Czerwinski D, Kwak LW, Liles TM, Syrengelas A, Taidi-Laskowski B and Levy R (1997) Tumor-specific idiotype vaccines in the treatment of patients with B-cell lymphoma--long-term results of a clinical trial. Blood 89: 3129-35.
Ivanov R, Hagenbeek A and Ebeling S (2006) Towards immunogene therapy of hematological malignancies. Exp Hematol 34: 251-63.
Kwak LW, Campbell MJ, Czerwinski DK, Hart S, Miller RA and Levy R (1992) Induction of immune responses in patients with B-cell lymphoma against the surface-immunoglobulin idiotype expressed by their tumors. N Engl J Med 327: 1209-15.
Kwak LW, Young HA, Pennington RW and Weeks SD (1996) Vaccination with syngeneic, lymphoma-derived immunoglobulin idiotype combined with granulocyte/macrophage colony-stimulating factor primes mice for a protective T-cell response. Proc Natl Acad Sci U S A 93: 10972-7.
Lim SH and Bailey-Wood R (1999) Idiotypic protein-pulsed dendritic cell vaccination in multiple myeloma. Int J Cancer 83: 215-22.
Lipford GB, Sparwasser T, Zimmermann S, Heeg K and Wagner H (2000) CpG-DNA-mediated transient lymphadenopathy is associated with a state of Th1 predisposition to antigen-driven responses. J Immunol 165: 1228-35.
Lotze MT, Shurin M, Esche C, Tahara H, Storkus W, Kirkwood JM, Whiteside TL, Elder EM, Okada H and Robbins P (2000) Interleukin-2: developing additional cytokine gene therapies using fibroblasts or dendritic cells to enhance tumor immunity. Cancer J Sci Am 6 Suppl 1: S61-6.
Massaia M, Borrione P, Battaglio S, Mariani S, Beggiato E, Napoli P, Voena C, Bianchi A, Coscia M, Besostri B, Peola S, Stiefel T, Even J, Novero D, Boccadoro M and Pileri A (1999) Idiotype vaccination in human myeloma: generation of tumor-specific immune responses after high-dose chemotherapy. Blood 94: 673-83.
Olerup O and Zetterquist H (1992) HLA-DR typing by PCR amplification with sequence-specific primers (PCR-SSP) in 2 hours: an alternative to serological DR typing in clinical practice including donor-recipient matching in cadaveric transplantation. Tissue Antigens 39: 225-35.
Osterborg A, Yi Q, Henriksson L, Fagerberg J, Bergenbrant S, Jeddi-Tehrani M, Ruden U, Lefvert AK, Holm G and Mellstedt H (1998) Idiotype immunization combined with granulocyte-macrophage colony-stimulating factor in myeloma patients induced type I, major histocompatibility complex-restricted, CD8- and CD4-specific T-cell responses. Blood 91: 2459-66.
Ramakrishna V, Vasilakos JP, Tario JD, Jr., Berger MA, Wallace PK and Keler T (2007) Toll-like receptor activation enhances cell-mediated immunity induced by an antibody vaccine targeting human dendritic cells. J Transl Med 5: 5.
Rasmussen T, Hansson L, Osterborg A, Johnsen HE and Mellstedt H (2003) Idiotype vaccination in multiple myeloma induced a reduction of circulating clonal tumor B cells. Blood 101: 4607-10.
Rechtsteiner G, Warger T, Osterloh P, Schild H and Radsak MP (2005) Cutting edge: priming of CTL by transcutaneous peptide immunization with imiquimod. J Immunol 174: 2476-80.
Reichardt VL, Okada CY, Liso A, Benike CJ, Stockerl-Goldstein KE, Engleman EG, Blume KG and Levy R (1999) Idiotype vaccination using dendritic cells after autologous peripheral blood stem cell transplantation for multiple myeloma--a feasibility study. Blood 93: 2411-9.
Rezvany MR, Jeddi-Tehrani M, Rabbani H, Ruden U, Hammarstrom L, Osterborg A, Wigzell H and Mellstedt H (2000) Autologous T lymphocytes recognize the tumour-derived immunoglobulin VH-CDR3 region in patients with B-cell chronic lymphocytic leukaemia. Br J Haematol 111: 230-8.
Romani N, Reider D, Heuer M, Ebner S, Kampgen E, Eibl B, Niederwieser D and Schuler G (1996) Generation of mature dendritic cells from human blood. An improved method with special regard to clinical applicability. J Immunol Methods 196: 137-51.
Shabani M, Asgarian-Omran H, Vossough P, Sharifian RA, Faranoush M, Ghragozlou S, Khoshnoodi J, Roohi A, Jeddi-Tehrani M, Mellstedt H, Rabbani H and Shokri F (2008) Expression profile of orphan receptor tyrosine kinase (ROR1) and Wilms' tumor gene 1 (WT1) in different subsets of B-cell acute lymphoblastic leukemia. Leuk Lymphoma 49: 1360-7.
Smith KJ, Hamza S and Skelton H (2004) Topical imidazoquinoline therapy of cutaneous squamous cell carcinoma polarizes lymphoid and monocyte/macrophage populations to a Th1 and M1 cytokine pattern. Clin Exp Dermatol 29: 505-12.
Smorlesi A, Papalini F, Orlando F, Donnini A, Re F and Provinciali M (2005) Imiquimod and S-27609 as adjuvants of DNA vaccination in a transgenic murine model of HER2/neu-positive mammary carcinoma. Gene Ther 12: 1324-32.
Sparwasser T, Vabulas RM, Villmow B, Lipford GB and Wagner H (2000) Bacterial CpG-DNA activates dendritic cells in vivo: T helper cell-independent cytotoxic T cell responses to soluble proteins. Eur J Immunol 30: 3591-7.
Sredni B and Schwartz RH (1981) Antigen-specific, proliferating T lymphocyte clones. Methodology, specificity, MHC restriction and alloreactivity. Immunol Rev 54: 187-223.
Sugai S, Palmer DW, Talal N and Witz IP (1974) Protective and cellular immune responses to idiotypic determinants on cells from a spontaneous lymphoma of NZB-NZW F1 mice. J Exp Med 140: 1547-58.
Sundaram R, Dakappagari NK and Kaumaya PT (2002) Synthetic peptides as cancer vaccines. Biopolymers 66: 200-16.
Thomsen LL, Topley P, Daly MG, Brett SJ and Tite JP (2004) Imiquimod and resiquimod in a mouse model: adjuvants for DNA vaccination by particle-mediated immunotherapeutic delivery. Vaccine 22: 1799-809.
Timmerman JM, Czerwinski DK, Davis TA, Hsu FJ, Benike C, Hao ZM, Taidi B, Rajapaksa R, Caspar CB, Okada CY, van Beckhoven A, Liles TM, Engleman EG and Levy R (2002) Idiotype-pulsed dendritic cell vaccination for B-cell lymphoma: clinical and immune responses in 35 patients. Blood 99: 1517-26.
Titzer S, Christensen O, Manzke O, Tesch H, Wolf J, Emmerich B, Carsten C, Diehl V and Bohlen H (2000) Vaccination of multiple myeloma patients with idiotype-pulsed dendritic cells: immunological and clinical aspects. Br J Haematol 108: 805-16.
Vonderheide RH, Hahn WC, Schultze JL and Nadler LM (1999) The telomerase catalytic subunit is a widely expressed tumor-associated antigen recognized by cytotoxic T lymphocytes. Immunity 10: 673-9.
Wagner TL, Ahonen CL, Couture AM, Gibson SJ, Miller RL, Smith RM, Reiter MJ, Vasilakos JP and Tomai MA (1999) Modulation of TH1 and TH2 cytokine production with the immune response modifiers, R-848 and imiquimod. Cell Immunol 191: 10-9.
Wen YJ, Ling M, Bailey-Wood R and Lim SH (1998) Idiotypic protein-pulsed adherent peripheral blood mononuclear cell-derived dendritic cells prime immune system in multiple myeloma. Clin Cancer Res 4: 957-62.
Yi Q, Desikan R, Barlogie B and Munshi N (2002) Optimizing dendritic cell-based immunotherapy in multiple myeloma. Br J Haematol 117: 297-305.

## Claims

1. Recombinant or synthetic 9-20 amino acid length peptide having amino acid sequence identity with an amino acid sequence selected from an expressed immunoglobulin variable region of a leukemic B cell, wherein said B cell is derived from a patient with a B cell malignancy, wherein said variable region is either:
a. the variable region of a IGHV consisting of an amino acid sequence starting at the first amino acid of framework 1 and ending at the last amino acid of HCDR3; or
b. the variable region of a IGLV consisting of an amino acid sequence starting at the first amino acid of framework 1 and ending at the last amino acid of LCDR3.

2. Recombinant or synthetic peptide of claim 1,
a. wherein said variable region of IGHV consists of an amino acid sequence starting at the first amino acid of framework 1 and ending at the last amino acid of HCDR2; and
b. wherein said variable region of IGLV consists of an amino acid sequence starting at the first amino acid of framework 1 and ending at the last amino acid of LCDR2.

3. Recombinant or synthetic peptide of claim 1,
a. wherein said variable region of IGHV consists of the amino acid sequence of HCDR3; and
b. wherein said variable region of IGLV consists of the amino acid of LCDR3.

4. Autologous dendritic cells pulsed with the recombinant or synthetic 9-20 amino acid length peptide of any one of claim 1 to 3.

5. A pharmaceutical composition comprising the recombinant or synthetic 9-20 amino acid length peptide of any one of claim 1 to 3, or the autologous dendritic cells of claim 4.

6. A pharmaceutical composition according to claim 5, further comprising an adjuvant.

7. A pharmaceutical composition according to claim 6, wherein said adjuvant is selected from TLR 7/8/9 agonists, GM-CSF, MPL.

8. A vaccine comprising the recombinant or synthetic 9-20 amino acid length peptide of any one of claims 1 to 3, or the pharmaceutical composition of any one of claims 5 to 7 for treatment of patients diagnosed with a B-cell malignancy.

9. A vaccine according to claim 8, wherein said B cell malignancy is selected from chronic lymphocytic leukemia (CLL), mantle cell lymphoma, hairy cell leukemia, diffuse large B-cell lymphoma, Burkitt's lymphoma, multiple myeloma and follicular lymphoma.

10. A vaccine according to claim 8 or 9, wherein said vaccine further comprises a carrier protein, wherein said peptide is unconjugated or conjugated with said carrier.

11. A vaccine according to claim 10, wherein said carrier is selected from tetanus toxoid or Id-keyhole-limpet hemocyanin.

12. A method of immunization of patients with B-cells malignancies (e.g. CLL) with the recombinant or synthetic peptide of any one of claims 1-3, or the pharmaceutical composition of any one of claims 4 to 7 for treatment of patients diagnosed with a B-cell malignancy, formulated as a vaccine, unconjugated or conjugated with a carrier protein such as tetanus toxoid,.

13. A method of immunization of patients with B-cells malignancies (e.g. CLL) with the autologous dendritic cells of claims 4.
